(19) Europäisches Patentamt
European Patent Office
Office européen des brevets

(11) Publication number: 0 003 738
A1

## (12) EUROPEAN PATENT APPLICATION

(21) Application number: 78300823.8

(22) Date of filing: 14.12.78

(51) Int. Cl.$^2$: C 07 C 49/36
C 07 C 43/18, C 07 C 97/07

(30) Priority: 14.12.77 JP 149347/77
28.12.77 JP 157326/77

(43) Date of publication of application:
05.09.79 Bulletin 79/18

(84) Designated contracting states:
DE FR GB NL

(71) Applicant: MITSUI PETRO CHEMICAL INDUSTRIES, LTD.
2-5, 3-chome Kasumigaseki Chiyoda-ku
Tokyo(JP)

(72) Inventor: Kiso, Yoshiha
2-3 Muroki-cho 1-chome
Iwakuni-shi Yamaguchi-ken(JP)

(72) Inventor: Saeki, Kenji
2-7, Misono 1-chome
Ohtake-shi Hiroshima-ken(JP)

(74) Representative: Myerscough, Philip Boyd (GB) et al.
J.A. Kemp & Co. 14 South Square, Gray's Inn
GB-London WC1R 5EU(GB)

# (54) Process for producing cyclohexane-1,3-diones or 3-alkoxy-2-cyclohexenones.

(57) 3-aminocyclohexene-2-one-1 is prepared by heating 5-oxohexanenitrile of the formula

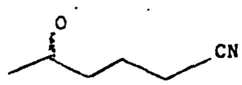

and cyclohexane-1.3-diones and 3-alkoxycyclohexene-2-ones-1 are prepared by reacting a 3-aminocyclohexene-2-one-1 of the formula

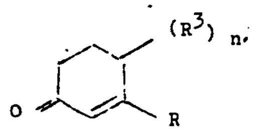

wherein R represents amino or alkylamino, $R^3$ represents a non-reactive substituent, and n is 0, 1 or 2, with water or a monohydric or dihydric alcohol of 1 to 10 carbon atoms.

The process provides the desired compounds, useful as intermediates in the preparation of medicines, agricultural chemicals, perfumes and resin substitutes, in good yields from readily available and inexpensive starting materials.

EP 0 003 738 A1

Croydon Printing Company Ltd.

DESCRIPTION

TITLE

"PROCESS FOR PRODUCING CYCLOHEXANE-1,3-DIONES OR 3-ALKOXY-2-CYCLOHEXENONES".

This invention relates to a process for producing cyclohexane-1,3-diones or 3-alkoxy-2-cyclohexenones in good yields with commercial advantage from relatively inexpensive and readily available materials.

Cyclohexane-1,3-diones or 3-alkoxy-2-cyclohexenones are useful as intermediates for the production of medicines, agricultural chemicals, perfumes, and stabilizers for synthetic resins. An especially useful application of the cyclohexane-1,3-diones is in the production of intermediates for resorcinol.

Japanese Laid-Open Patent Publication No. 69633/74 (corresponding to German OLS P 2245270.6) discloses a method for producing cyclohexane-1,3-diones which comprises reacting a γ-acylcarboxylic acid ester with a strongly basic condensing agent in a solvent such as a carboxylic acid amide, a phosphoric acid amide, a sulfoxide or sulfone, or a macrocyclic polyether.

Japanese Laid-Open Patent Publication No. 29532/75 (corresponding to Dutch Patent Application No. 7303536) discloses a process for producing cyclohexane-1,3-dione or its alkyl-substituted derivatives by contacting an alkyl 5-oxohexanoate at about 100 to 500°C with a solid material having a large inside surface area such as activated carbon, magnesium oxide, barium oxide or graphite in the gaseous phase.

Japanese Laid-Open Patent Publication No. 68546/76 (corresponding to German OLS P 2448677.9) discloses a process for producing a mixture of cyclohexane-1,3-dione and 6-alkyl-3,4-dihydro-2-pyranone by cyclizing 5-oxohexanoic acid in the vapor phase at 250 to 500°C in the presence of a weakly basic or weakly acidic dehydration catalyst.

Japanese Laid-Open Patent Publication No. 17446/77 (corresponding to German OLS P 2533919.9) discloses a process for producing cyclohexane-1,3-dione, which comprises reacting an α,β-unsaturated carboxylic acid ester with a ketone in the presence of a strong base in a specified carboxylic acid amide, phosphoric acid amide, sulfoxide, sulfone or ether as a solvent.

These known processes have one or more disadvantages. For example, the starting materials are expensive and are difficult to obtain, the reaction conditions are disadvantageous; the reaction does not proceed smoothly; the reaction operation is complicated; side-reactions tend to take place; expensive solvents are required; or the yield is unsatisfactory.

The invention provides a process wherein cyclohexane-1,3-diones or 3-alkoxy-2-cyclohexenones can be obtained in good yields with commercial advantage from inexpensive starting materials under mild reaction conditions and without using any special solvent.

The invention thus provides a process for producing cyclohexane-1,3-diones or 3-alkoxy-2-cyclohexenones of the formula

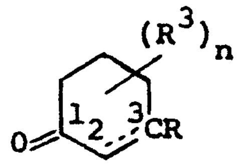

(1)

wherein R represents =O or $-OR^2$, in which when R is =O, the bond between the 2- and 3-positions is a carbon-carbon single bond, and when R is $-OR^2$, the bond between the 2- and 3-positions is a carbon-carbon double bond; $R^2$ represents a residual moiety of a monohydric or dihydric alcohol having 1 to 10 carbon atoms; $R^3$ represents a substituent which is non-reactive under the reaction conditions; and n is 0, 1 or 2, which comprises reacting a 3-(substituted or unsubstituted amino)-2-cyclohexenone of the formula

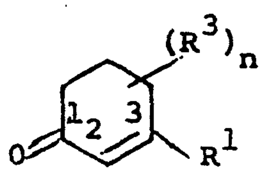

(2)

wherein $R^1$ repreeents an amino group or an amino group substituted by a linear, branched or cyclic alkyl group having 1 to 6 carbon atoms.

The process of the invention, involves hydrolysis or alcoholysis of the 3-(substituted or unsubstituted amino)-2-cyclohexenones of formula (2), which is quite a different mode of reaction from those suggested heretofore, and can easily give the corresponding

cyclohexane-1,3-diones or 3-alkoxy-2-cyclohexenones of formula (1). Production of the compounds of formula (1) from the compounds of formula (2) by the new mode of reaction in accordance with the process of this invention is schematically illustrated below.

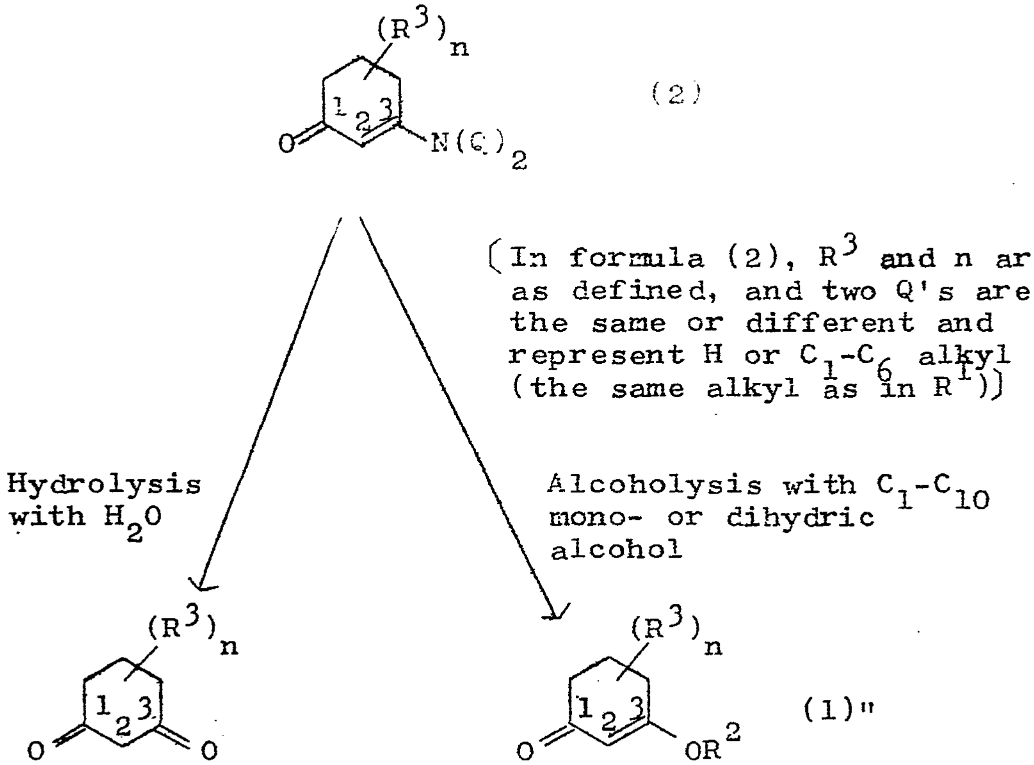


(2)

[In formula (2), $R^3$ and n are as defined, and two Q's are the same or different and represent H or $C_1$-$C_6$ alkyl (the same alkyl as in $R^1$)]

(1)'

(1)"

[In formula (1)', $R^3$ and n are as defined in formula (2)]

[In formula (1)", $R^2$ is as defined in formula (1), and $R^3$ and n are as defined in formula (2)]

As illustrated, according to the process of this invention, the cyclohexane-1,3-diones of formula (1)' can be obtained by the hydrolysis reaction with water, and the 3-alkoxy-2-cyclohexenones of formula (1)" can be obtained by the alcoholysis reaction with the alcohol.

In the compound of formula (2) above, $R^3$ is any substituent which is non-reactive under the reaction conditions. Examples are lower alkyl groups with 1 to 4 carbon atoms, lower alkoxy groups with 1 to 4 carbon atoms, aromatic groups with 6 to 10 carbon atoms, a hydroxyl group, a nitro group, and halogen atoms such as chloro.

Specific examples of the compound of formula (2) include 3-amino-2-cyclohexenone, 3-amino-2-methyl-2-cyclohexenone, 3-amino-5-methyl-2-cyclohexenone, 3-amino-5,5-dimethyl-2-cyclohexenone, 3-amino-2,6-dimethyl-2-cyclohexenone, 3-amino-4-phenyl-2-cyclohexenone, 3-amino-5-phenyl-2-cyclohexenone, 3-amino-6-phenyl-2-cyclohexenone, 3-amino-2-bromo-5,5-dimethyl-2-cyclohexenone, and 3-amino-2-nitro-5,5-dimethyl-2-cyclohexenone. Compounds resulting from the substitution of N-mono-substituted or N-di-substituted-$C_1$-$C_6$ alkylamino groups for the amino group of above-illustrated compounds can also be used. Examples of such substituted amino groups include N-methylamino, N-ethylamino, N-isopropylamino, N-n-butylamino, N-t-butylamino, N-cyclohexylamino, N,N-dimethylamino, N,N-diethylamino, and N,N-dibutylamino groups.

Examples of the $C_1$-$C_{10}$ mono- or dihydric alcohols used in the practice of the process of this invention include methanol, ethanol, n-propanol, n-butanol, isopropanol, sec-butanol, cyclohexanol, ethylene glycol, diethylene glycol, and benzyl alcohol. Of these, the monohydric primary lower alcohols such as methanol or ethanol are especially preferred.

The reaction is suitably carried out at a temperature of, say, about 0° to about 200°C, preferably from room temperature to about 150°C. In the reaction, at least 1 mole of the water or the $C_1$-$C_{10}$ mono- or dihydric alcohol is used per mole of the starting compound of formula (2). For example, the water or alcohol is used in an amount of about 1 to about 100 moles per mole of the compound of formula (2). If desired, a solvent can be used in the reaction, but is not essential in the process of this invention. Rather, the absence of solvent is preferred to facilitate the operation of separating the desired product. Usable solvents may include benzene and toluene.

The reaction time can be varied according to the reaction temperature and the use or non-use of catalyst.

For example, it is from about 0.1 to about 10 hours.

The rate of the reaction in the process of this invention can be greatly increased by performing the reaction in the presence of a catalyst, and this is preferred because the selectivity for the cyclohexane-1,3-diones or 3-alkoxy-2-cyclohexenones can be increased. Basic catalysts and acid catalysts are preferred catalysts. Examples of the basic catalysts include alkali metal hydroxides such as lithium hydroxide, sodium hydroxide and potassium hydroxide; alkali metal oxides such as lithium oxide, sodium oxide and potassium oxide; alkali metal alkoxides such as lithium methoxide, lithium ethoxide, sodium methoxide, sodium ethoxide, sodium propoxide, potassium ethoxide, potassium isopropoxide and potassium butoxide; alkaline earth metal hydroxides such as magnesium hydroxide; calcium hydroxide, strontium hydroxide and barium hydroxide; and basic organic compounds such as trimethylamine, triethylamine, tri-n-propylamine, triisopropylamine, tetramethyl ammonium hydroxide, tetraethyl ammonium hydroxide, trimethyl benzyl ammonium hydroxide, triethyl benzyl ammonium hydroxide, triisopropyl benzyl ammonium hydroxide and diazabicycloundecane. Of these basic catalysts, the alkali metal-containing basic compounds are especially preferred. Examples of the acid catalyst are inorganic compounds such as hydrochloric acid, hydrobromic acid, hydriodic acid, sulfuric acid, nitric acid, perchloric acid, phosphoric acid, monophosphates, diphosphates, carbonic acid, carbon dioxide and ammonium hydrogen sulfate; and organic compounds such as formic acid, acetic acid, monochloroacetic acid, dichloroacetic acid, trichloroacetic acid and p-toluenesulfonic acid. Of these acid catalysts, strongly acid compounds such as sulfuric acid and hydrochloric acid are especially preferred.

The amount of the catalyst is not critical, and may, for example, be about 0.1 to about 10 gram-equivalents, preferably about 1 to about 2 gram-equivalents,

per mole of the 3-(substituted or unsubstituted amino)-2-cyclohexenone.

After the reaction, the compound of formula (1) can be separated by applying such means as distillation or crystallization to the reaction mixture.

The starting 3-(substituted or unsubstituted amino)-2-cyclohexenone used in the performance of this invention may be produced by any desired process. For example, it can be produced by the process disclosed in Japanese Patent Publication No. 34859/73 (corresponding to German OLS 2144169 and 2144170) which comprises cyclizing a β-cyanoethylated ketone in a polar solvent in the presence of a basic catalyst at a temperature of about 20° to about 150°C. In particular, 3-amino-2-cyclohexenone can be produced in good conversion and selectivity by the following process developed by the present inventors which involves the heat cyclization of 5-oxohexanenitrile which is readily synthesized from acetone and acrylonitrile. Specifically this process comprises heating 5-oxohexanenitrile of the formula

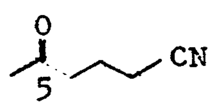

at a temperature of about 160°C to about 220°C in the presence of a basic catalyst in an inert organic polar solvent. Specific examples of the solvent used in this process include alcohols containing 1 to 6 carbon atoms such as methanol, ethanol, n-propanol, isopropanol, n-butanol, sec-butanol, tert-butanol, pentanol, tert-amylalcohol, and hexanol; ethers containing 4 to 14 carbon atoms such as diethyl ether, tetrahydrofuran, dioxane, diglyme and triethylene glycol dimethyl ether; nitriles containing 1 to 3 carbon atoms such as acetonitrile and propionitrile; amines containing 3 to 8 carbon atoms such as trimethylamine, triethylamine, pyridine and α-picoline; dialkylformamides with the alkyl group containing 1 or 2 carbon atoms such as dimethylformamide and diethylformamide;

phosphoric acid amides such as hexamethylphosphoric triamide; and sulfoxides such as dimethyl sulfoxide. Of these, tertiary alcohols, especially tert-butanol, are preferred. Mixtures of two or more of these organic polar solvents, and mixtures of them with organic non-polar solvents can also be used. Examples of the organic non-polar solvents are aliphatic hydrocarbons such as hexane, heptane, octane, nonane and decane; and aromatic hydrocarbons such as benzene, toluene and p-xylene. The amount of the organic polar solvent can be used in an amount such that the weight ratio of it to the 5-oxohexanenitrile is from 1:1 to 1000:1, preferably from 5:1 to 200:1.

Specific examples of the basic catalyst used in the above process are alkali metal hydroxides such as potassium hydroxide and sodium hydroxide; alkali metal alkoxides such as potassium methoxide, potassium ethoxide, potassium propoxides and potassium butoxides and the corresponding sodium alkoxides; alkali metal amides such as sodium amide and potassium amide; alkali metal hydrides such as sodium hydride; and alkali metals such as sodium and potassium. Of these basic catalysts, sodium hydroxide, potassium hydroxide, potassium alkoxides, and sodium alkoxides are preferred. Potassium hydroxide and potassium alkoxides are especially preferred. The amount of the basic catalyst can be varied widely. Usually, it is about 0.001 to about 1 mole, preferably about 0.005 to about 0.2 mole, per mole of the starting 5-oxohexanenitrile.

The heat cyclization reaction is carried out at a temperature of about 160 to about 220°C, preferably about 170 to about 200°C. If the heating temperature is lower than about 160°C, the conversion of the 5-oxohexanenitrile decreases, and its selectivity for 6-methyl-3,4-dihydro-2-pyridone, a by-product, increases abruptly, thus causing a reduction in the selectivity for 3-amino-2-cyclohexanone and its yield. On the other hand, if the heating temperature exceeds about 220°C, the selection for the by-product 6-methyl-3,4-dihydro-2-pyridone

increases, and 3-amino-2-cyclohexenone which has been formed may be consumed by a secondary reaction. Hence, the selection for 3-amino-2-cyclohexenone and its yield decrease. By maintaining the heating temperature at 170 to 200°C, the selectivity for 3-amino-2-cyclohexenone and its yield can be increased further.

The above process can be performed either batchwise, semicontinuously or continuously. When 5-oxohexanenitrile is fully diluted with a solvent in the aforesaid process, the formation of the by-product 6-methyl-3,4-dihydro-2-pyridone can be inhibited to some extent, and the selectivity for 3-amino-2-cyclohexenone can be increased. Accordingly, it is preferred to use the solvent in an amount which is as excessive as is commercially permissible. It is preferred that the reaction be carried out under conditions such that the concentration of the starting 5-oxohexanenitrile in the reaction system does not exceed about 0.5 mole/liter, for example, under such conditions that the concentration of the 5-oxohexanenitrile is about 0.1 to about 0.5 mole/liter of solvent. The reaction time is not critical, either, and may, for example, be about 0.5 to about 4 hours.

The selectivity for 3-amino-2-cyclohexenone can thus be increased if 5-oxohexanenitrile is added to the reaction system so that the concentration of the unreacted 5-oxohexanenitrile in the reaction solution does not exceed 0.5 mole/liter.

After the reaction, the reaction mixture is treated in a customary manner such as distillation or crystallization to separate 3-amino-2-cyclohexenone.

Thus, according to one preferred embodiment of this invention, there is provided a process for producing a cyclohexane-1,3-dione or 3-alkoxy-2-cyclohexenone of the formula

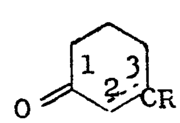

wherein R represents =O or $-OR^2$ in which when R is

=O, the bond between the 2- and 3-positions represents a carbon-carbon single bond, and when R is $-OR^2$, the bond between the 2- and 3-positions represents a carbon-carbon double bond; and $R^2$ represents a residual moiety of a monohydric or dihydric alcohol having 1 to 10 carbon atoms,

which comprises heating 5-oxohexanenitrile of the formula

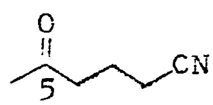

at a temperature of about 160°C to about 220°C in the presence of a basic catalyst in an inert organic polar solvent to form 3-amino-2-cyclohexenone of the formula

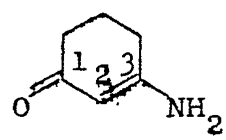

and reacting the resulting 3-amino-2-cyclohexenone with water or a monohydric or dihydric alcohol having 1 to 10 carbon atoms.

The following Examples illustrate the process of this invention in greater detail.

Material Production Examples 1 and 2

A 50 ml stainless steel autoclave purged with argon was charged with 5-oxohexanenitrile, potassium t-butoxide and t-butanol in the amounts indicated in Table 1. The reaction was performed under the conditions shown in Table 1. After the reaction, the reaction mixture was gas-chromatographically analyzed to determine the conversion of 5-oxohexanenitrile, and its selectivity for 3-amino-2-cyclohexenone and for the by-product 6-methyl-3,4-dihydro-2-pyridone. The results are shown in Table 1.

Table 1

| Material Production Example | 1 | 2 |
|---|---|---|
| Amount of 5-oxohexanenitrile (mmoles) | 9.30 | 8.89 |
| Amount of potassium t-butoxide (mmoles) | 0.85 | 0.87 |
| Amount of t-butanol (ml) | 30 | 30 |
| Reaction temperature (°C) | 180 | 200 |
| Reaction time (hours) | 3 | 3 |
| Conversion of 5-oxohexanenitrile (%) | 97 | 95 |
| Selectivity (%) for | | |
| 3-Amino-2-cyclohexenone | 80 | 78 |
| 6-Methyl-3,4-dihydro-2-pyridone | 15 | 17 |

Examples 1 to 18

A 100 ml three-necked flask equipped with a stirrer and a condenser was charged with the 3-amino-2-cyclohexenone prepared in Material Production Example 1, each of the catalysts indicated in Table 2 and water, and the reaction was performed under the reaction conditions shown in Table 2. After the reaction, the amounts of the unreacted 3-amino-2-cyclohexenone, cyclohexane-1,3-dione and δ-ketocaproic acid and other by-products in the resulting reaction mixture were determined by gas chromatography. From the results obtained, the conversion of 3-amino-2-cyclohexenone and its selectivity for cyclohexane-1,3-dione were calculated. The results are shown in Table 2. When the basic catalyst was added, the reaction mixture was neutralized with sulfuric acid before the gas-chromatographic analysis.

**Table 2**

| Ex-ample | 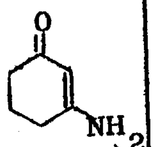 (mmol) | Water (ml) | Catalyst (mg-equivalent) | Reaction conditions: Tempe-rature (°C) | Reaction conditions: Time (hr) | Results of the reaction: 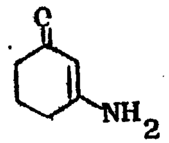 Conversion (%) | Selectivity (%) for: 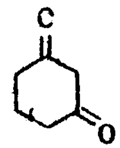 | Selectivity (%) for: 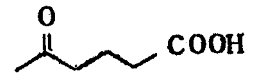 | Selectivity (%) for: Other by-products |
|---|---|---|---|---|---|---|---|---|---|
| 1 | 8.2 | 1.5 | Not added (——) | 100 | 4.5 | 7 | 68 | 29 | 4 |
| 2 | " | "(*1) | " ( " ) | 170 | 2.5 | 10 | 66 | 31 | 3 |
| 3 | 32.3 | 8.0 | HCl (0.3) | 100 | 1.5 | 10 | 96 | 4 | 0 |
| 4 | " | 11.0 | " (0.6) | 100 | 2.0 | 15 | 88 | 11 | 1 |
| 5 | " | " | " (0.9) | 100 | 3.0 | 16 | 84 | 15 | 1 |
| 6 | 17.6 | 12 | " (17.5) | RT(*2) | 1.5 | 37 | 100 | 0 | 0 |
| 7 | " | " | " ( " ) | " | 5.0 | 56 | 100 | 0 | 0 |
| 8 | " | " | " ( " ) | " | 24.0 | 80 | 100 | 0 | 0 |
| 9 | 15.9 | " | " (15.9) | 80 | 0.3 | 80 | 99 | 0 | 1 |
| 10 | " | " | " ( " ) | " | 0.7 | 84 | 97 | 0 | 3 |
| 11 | " | " | " ( " ) | " | 1.3 | 85 | 98 | 0 | 2 |
| 12 | 23.8 | 4 | $H_2SO_4$ (24.0) | " | 2.0 | 96 | 98 | 0 | 2 |
| 13 | 22.8 | 14 | " (23.0) | " | 1.0 | 66 | 100 | 0 | 0 |
| 14 | 13.5 | 8 | $CH_3COOH$ (13.5) | RT | 1.8 | 15 | 100 | 0 | 0 |
| 15 | " | " | " ( " ) | " | 5 | 20 | 100 | 0 | 0 |
| 16 | " | " | " ( " ) | " | 24 | 29 | 100 | 0 | 0 |
| 17 | 25.0 | 11 | $NH_4HSO_4$ (25.0) | 80 | 0.3 | 48 | 100 | 0 | 0 |
| 18 | 16.5 | 19 | KOH (34.2) | 100 | 1.5 | 99 | 89 | 11 | 0 |

(*1): 3 ml of triethylene glycol dimethyl ether was added, and the mixture was heated.

(*2): RT = room temperature.

Examples 19 and 20

A 50 ml stainless steel autoclave was charged with 3-amino-2-cyclohexenone (prepared in Material Production Example 1) and water in the amounts indicated in Table 3, and the pressure in the autoclave was raised to 50 $kg/cm^2$ by introducing carbon dioxide gas. The reaction was carried out under the conditions shown in Table 3. After the reaction, the reaction mixture was analyzed in the same way as in Example 1. The results are shown in Table 3.

Table 3

| Example | 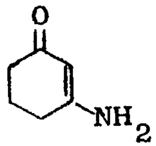 (mmol) | Water (ml) | Reaction conditions | | Results of the reaction | | | |
|---|---|---|---|---|---|---|---|---|
| | | | Tempe-rature (°C) | Time (hr) | 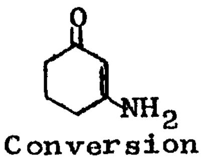 Conversion (%) | Selectivity (%) for | | |
| | | | | | | 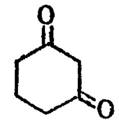 | 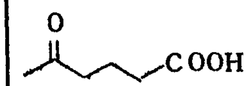 | Other by-products |
| 19 | 11.5 | 10 | Room tempe-rature | 5 | 17 | 100 | 0 | 0 |
| 20 | " | " | 100 | 2 | 29 | 100 | C | 0 |

Example 21

A flask was charged with 7.75 mmoles of 3-N,N-diethylamino-2-cyclohexenone, 3.0 ml of water and 1.2 ml (7.8 mg equivalents) of 6.55N sulfuric acid, and the mixture was heated for 4 hours at 80°C. After the reaction, the reaction mixture was analyzed by gas-chromatography in the same way as in Example 1. It was found that the conversion of 3-diethylamino-2-cyclohexenone was 74%, and its selectivity for cyclohexane-1,3-dione was 100%.

Example 22

A mixture of 45.6 mmoles of 3-amino-2-cyclohexenone, 422.5 mmoles of methanol and 44.2 mg equivalents of 97% sulfuric acid was heated at 100°C for 2 hours. After the reaction, chloroform was added, and the mixture was filtered. The filtrate was concentrated, and extracted with benzene. The extract was concentrated to afford 3.44 g (27.3 mmoles; yield 60%) of 3-methoxy-2-cyclohexenone.

Example 23

A mixture of 44.9 mmoles of 3-amino-2-cyclohexenone, 439.0 mmoles of isopropanol and 47.3 mg equivalents of 97% conc. sulfuric acid was heated at 100°C for 2 hours. After the reaction, the reaction mixture was gas-chromatographically analyzed. It was found that 2.6% of 3-isopropoxy-2-cyclohexenone was formed.

## CLAIMS

1. A process for producing cyclohexane-1,3-diones or 3-alkoxy-2-cyclohexenones of the formula

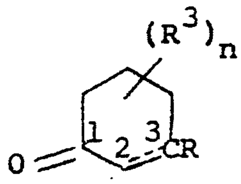

(1)

wherein R represents =O or $OR^2$, in which when R is =O, the bond between the 2- and 3-positions is a carbon-carbon single bond, and when R is $-OR^2$, the bond between the 2- and 3-positions is a carbon-carbon double bond; $R^2$ represents a residual moiety of a monohydric or dihydric alcohol having 1 to 10 carbon atoms; $R^3$ represents a substituent which is non-reactive under the reaction conditions; and n is 0, 1 or 2,

which process comprises reacting a 3-(substituted or unsubstituted amino)-2-cyclohexenone of the formula

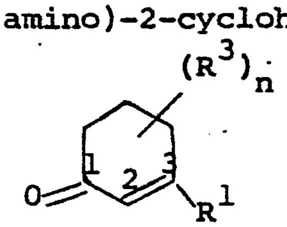

(2)

wherein $R^1$ represents an amino group or an amino group substituted by a linear, branched or cyclic alkyl group having 1 to 6 carbon atoms, and $R^3$ and n are as defined above,

with water or a monohydric or dihydric alcohol having 1 to 10 carbon atoms.

2. A process according to claim 1 wherein the non-reactive substituent $R^3$ is a member selected from alkyl or alkoxy groups, of 1 to 4 carbon atoms, aromatic groups of 6 to 10 carbon atoms, a hydroxyl group, a nitro group and halogen atoms.

3. A process according to claim 1 or 2 wherein the reaction is carried out in the presence of a basic or acidic catalyst.

4. A process according to claim 3 wherein the amount of the catalyst is 0.1 to 10 gram-equivalents

per mole of the 3-(substituted or unsubstituted amino)-2-cyclohexenone.

5. A process according to any one of claims 1 to 4 wherein the reaction is carried out at a temperature of 20° to 150°C.

6. A process according to any one of claims 1 to 4 wherein the compound of formula (2) is 3-amino-2-cyclohexenone of the formula

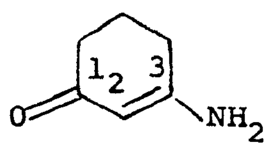
(2)

which has been produced by heating 5-oxohexanenitrile of the formula

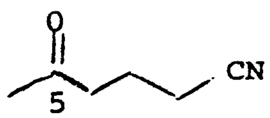

at a temperature of 160° to 220°C in the presence of a basic catalyst in an inert organic polar solvent.

0003738

European Patent Office

# EUROPEAN SEARCH REPORT

EP 78 [illegible]

## DOCUMENTS CONSIDERED TO BE RELEVANT

| Category | Citation of document with indication, where appropriate, of relevant passages | Relevant to claim |
|---|---|---|
| X | FR - A - 2 151 105 (DEUTSCHE TEXACO)<br>* claim 1, page 1, lines 29 to 30 *<br>-- | 5 |
| | JP - B - 66-2617 (KYOWA FERMENTATION)<br>& Chemical Abstracts vol. 64, no. 10 1966<br>Columbus, Ohio, USA<br>M. TANAKA et al "α -Dicarbonyl compounds"<br>column 14134 h<br>-- | 1 |
| A | GB - A - 879 564 (H.J. ZIMMER)<br>* claim 1 *<br>---- | 1 |

CLASSIFICATION OF THE APPLICATION (Int. Cl.)

C 07 C 45/36
C 07 C 43/13
C 07 C 97/07

TECHNICAL FIELDS SEARCHED (Int. Cl.)

C 07 C 41/00
C 07 C 43/13
C 07 C 45/00
C 07 C 49/30
C 07 C 49/36
C 07 C 49/60
C 07 C 85/12
C 07 C 97/07

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 13-03-1979 | KNAACK |

EPO Form 1503.1 06.78